# EUROPEAN PATENT APPLICATION

(11) **EP 2 656 875 A1**
(43) Date of publication of application: **30.10.2013**
(21) Application number: 12165421.4
(22) Date of filing: 25.04.2012
(51) Int. Cl.: A61N 1/37, A61N 1/08

(54) **Lead for a neurostimulation and/or neurorecording system**

(71) Applicant: Sapiens Steering Brain Stimulation B.V., 5656 AE Eindhoven (NL)
(72) Inventor: Young, Edward Willem Albert, 6211 LN Maastricht (NL); Bakker, Egbertus Johannes Maria, 4261 BX Wijk en Aalburg (NL); Harberts, Dick Willem, 5627 MZ Eindhoven (NL)
(74) Representative: Rupprecht, Kay

(57) **Abstract**

The present invention relates to a lead (300), especially a lead (300) for neural applications, preferably a lead (300) for a neurostimulation and/or neurorecording system, whereby the lead (300) comprises a proximal end (311) and a distal end (313) and whereby the lead (300) comprises at least one extension means (320) at the distal end (313). Furthermore, the present invention relates to a probe and a system (100), especially a neurostimulation and/or neurorecording system (100).

## Description

The present invention relates to a lead, especially to a lead for a neurostimulation and/or neurorecording system.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylus material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

Typically, the MR compatibility of probes is an issue that should be addressed. Serious field enhancement may occur as a result of the electrical field during scanning. The high field gives rise to tissue heating. It is common practice to coil the lead wires to create inductance. The inductance limits the creation of strong electric fields in the brain during MR scanning and subsequent heating in these areas of the brain. However, field gradient enhancement near the probe, in particular at the distal-end, is still an issue, also because the distal-end of the electrode is not coiled and therefore it is not an integral part of the inductor. Moreover, the field at the probe tip is dominant because the enhancement gradient is most pronounced in this area and most heating of tissue is expected at location in close vicinity of the distal-end in particular.

Heating of the tissue as a result of high electric field strength near the probe during MRI scanning is a serious risk. In particular, strong fields may be generated at the lead distal end.

It is therefore an object of the present invention, to improve a lead, a probe, and a neurostimulation and/or neurorecording system, particularly in that the heating of the tissue as a result of high electric fields strength near the lead during MRI scanning is lowered.

The above object is solved according to the present invention by a lead according to claim 1. Accordingly, a lead is provided, whereby the lead comprises a proximal end and a distal end and whereby the lead comprises at least one extension means at the distal end.

The lead may be e.g. a lead for neural applications, preferably a lead for a neurostimulation and/or neurorecording system. The neurostimulation and/or neurorecording system can be e.g. a DBS system. Generally, the lead may be adapted for brain application.

The distal end of the lead may be the end of the lead, which is in the implanted state of the lead the remote end of the lead with regard to the body surface area. In particular, in case of a lead for brain application, the distal end of the lead is the lower end of the lead, which remote to the burr-hole of the skull, through which the lead is implanted.

According to the present invention a modification and reduction of the electric-field enhancement in the close vicinity of the distal end is achieved by the design of the distal end of the lead. The present invention targets the field around the lead tip. To this end, an extension is added on the lead, e.g. a thin film based lead, to reduce the enhancement of the electrical field at the lead tip. Thereby, heating of the tissue as a result of high electric fields strength near the lead during MRI scanning is lowered due to the by fact that by adding an extension means to the distal end of the thin film due to the fact that the resulting density of equipotential lines can be lowered by the extension means.

Moreover, it is possible that the lead comprises a lead tip at the distal end and a plurality of electrodes arranged at the distal end of the lead and that the extension means is arranged between the plurality of electrodes and the lead tip.

Additionally, it is possible that the extension means has a length of at least 1 mm or more, especially 1.5 mm or more, preferably 2 mm or more. Preferably, the extension means may be made of a polymer. The extension means may have a dielectric constant ε of around 3. It has been surprisingly shown that an extension of the distal end significantly reduces the accumulation of electric field lines near the tip end. Such an extension should be e.g. an extension of the space between the electrodes and the distal lead tip having a length of at least 1 mm, especially 1.5 mm or more, preferably 2 mm or more.

The extension means may be formed separately from the lead. In particular, the extension means may be a separate part which is connected to the distal end of the lead. Further, the extension means may at least partially include a different material than the distal end of the lead. The extension means may form the lead tip.

By this, the effect is achieved that a high field concentration occurs in the extension means. An extension means with a dielectric constant which is configured such that the accumulation of electric field lines near the lead tip is reducible or reduced can be provided by using at least partially a material for the extension means that has a relatively low dielectric constant ε, e.g. a low permittivity material such as a polymer. Thereby, the accumulation of electric field lines near the tip end can be significantly reduced. The high field concentration will occur in the low permittivity extension material. There, it does not give rise to heating.

Furthermore, it is possible that the extension means has a dielectric constant which is configured such that the accumulation of electric field lines near the lead tip is reducible or reduced.
With a higher dielectric constant material, such as Ba-titanate, the effect of reduced accumulation of field lines near the probe tip can be realised with a smaller extension.

It is also possible that the extension means comprises at least partially a resistive material having an ohmic resistivity. Also an Ohmic resistance, the effect of reduced accumulation of field lines near the probe tip can be realised with a relatively smaller extension. The resistivity of the extension should be in the order of 10 S/m.

The resistivity may be relatively large, preferably 10 S/m at maximum. Such a high Ohmic core material can be manufactured from polymer mixtures with high Ohmic materials such as sub-stoichiometric powder of Siliconoxide, Silicon nitride or Titanium-oxide. In particular, the resistivity is selected at a value which is at least larger than the resistivity of a material used for conducting current and thus having a good electrical conductivity and a low resistivity. By using a resistive material having an ohmic resistivity for the extension means, the field gradient induced during magnetic resonance imaging at the distal end of the lead can be decreased. By providing an extension means adjacent to the distal tip end of the lead the equipotential lines of the field being existent during magnetic resonance imaging can cross the region of the resistive material having an ohmic resistivity, which is not the case when there is no region with relatively high resistivity. Since these equipotential lines do not have to bend around the tip of the lead, the resulting density of equipotential lines can lowered. This results in lower electric field strength at the tip end of the lead and thus the risk of increasing the temperature at the tip end is significantly lowered.

Moreover, it is possible that the extension means is configured such that it yields impedance, Z, when exposed to frequencies used during magnetic resonance imaging. Thereby, a similar effect can be achieved like using an extension means comprising at least partially a resistive material having an ohmic resistivity. Similarly, thereby the field gradient induced during magnetic resonance imaging at the distal end of the lead can be decreased. Since the equipotential lines do not have to bend around the tip of the lead, the resulting density of equipotential lines can lowered. This results in lower electric field strength at the tip end of the lead and thus the risk of increasing the temperature at the tip end is significantly loweredIt is possible that the extension means comprises at least partially particles of conducting material, e.g. nano materials, especially carbon black, whereby preferably the extension means comprises a polymer matrix carrying and/or enclosing the carbon black to create impedance, Z. Such particles of conducting material may be e.g. dispersed in an insulating material, such as e.g. a polymer. The conducting material may be e.g. chosen from a group of conducting materials which are able to provide percolation. The concentration of the conducting material may be chosen such that the loss of energy of a radio frequency signal is significant or significantly high within a frequency interval covering the frequency of an electro magnetic radio frequency field originating from a magnetic resonance imaging apparatus. So, e.g. by the use of nano materials like carbon black dispersed within a polymer matrix, a so-called "high-loss" impedance material can be provided.

Moreover, it is possible that the extension means comprises at least partially a resistive section having a capacitive resistivity and/or that the extension means comprises at least one capacitive coupling means having a capacitive resistivity. Thereby, a similar effect can be achieved like using an extension means comprising at least partially a resistive material or an extension means which is configured such that it yields impedance when exposed to frequencies used during magnetic resonance imaging. Similarly, thereby the field gradient induced during magnetic resonance imaging at the distal end of the lead can be decreased and the resulting density of equipotential lines can lowered. This results also in lower electric field strength at the tip end of the lead and thus the risk of increasing the temperature at the tip end is significantly lowered. Particularly, it is possible that the extension means comprises a plurality of capacitors. Capacitive coupling of the capacitor to the distal and coupeling between subsequent capacitors should be in the order of 20 pF.

Generally, it is possible to combine several or all of the above possibilities to increase the resistivity and/or impedance of the extension means to decrease the field gradient induced during magnetic resonance imaging at the distal end of the lead. In fact, the advantageous effect is not limited to ohmic resistors alone, but can be achieved by any structure that yields impedance at MR frequencies. Moreover, the high frequency impedance can also be realized as a capacitor.

Additionally, it is possible that the extension means is arranged and/or configured such that it comprises at least partially a resistivity and/or impedance gradient. By providing a gradient, the effectiveness of a resistive extension means to the distal end of the lead can be further enhanced.

Furthermore, it is possible that the resistivity and/or impedance gradient is configured such that the extension means comprises at least a first resistivity and/or impedance at the one end of the extension means and at least a second resistivity and/or impedance at a second end of the extension means, whereby the first resistivity and/or impedance is higher than the second resistivity and/or impedance.

Moreover, it is possible that the first end of the extension means is arranged near to the distal tip of the lead and the second end of the extension means is arranged next to the electrodes. This alignment may advantageously prevent the development of strongly enhanced gradients of the field at the side of the probe, near to the electrode area.

It is possible that the lead tip is a blunted polymer stylus tip.

Additionally, the present invention relates to a probe with the features of claim 14. Accordingly, a probe is provided, which comprises at least one lead according to any of claims 1 to 13. Especially, the probe may be a probe for brain applications, preferably a probe for a neurostimulation and/or neurorecording system. Such a probe may comprise a lead and an Advanced Lead Connector (ALC) element comprising electronic means to address electrodes on the distal end of the lead.

Furthermore, the present invention relates to a neurostimulation and/or neurorecording system with the features of claim 15. Accordingly, a neurostimulation and/or neurorecording system is provided, which is comprising at least one lead according to any of claims 1 to 13 and/or a probe according to claim 14. The neurostimulation and/or neurorecording system may be especially a deep brain stimulation (DBS) system.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a schematical drawing of the field enhancement around a conducting lead in a brain in an electric field during an MRI scan;
- Fig. 5:: a diagram regarding the relation between the electric field enhancement at 64 MHz;
- Fig. 6:: a schematical drawing showing the field enhancement around a conducting body with varying resistivity in an electric field;
- Fig. 7a, b:: schematical drawings regarding the field distribution around a lead in an electric field during an MRI scan;
- Fig. 8:: a schematical drawing of the distal section of a known lead;
- Fig. 9:: a schematical drawing of the distal section of a further known lead;
- Fig. 10:: a schematical drawing of a first embodiment of the present invention;
- Fig. 11:: a schematical drawing of a second embodiment of the present invention;
- Fig. 12:: a schematical drawing of a third embodiment of the present invention;
- Fig. 13:: a schematical drawing of a fourth embodiment of the present invention; and
- Fig. 14:: a schematical drawing of a fifth embodiment of the present invention.

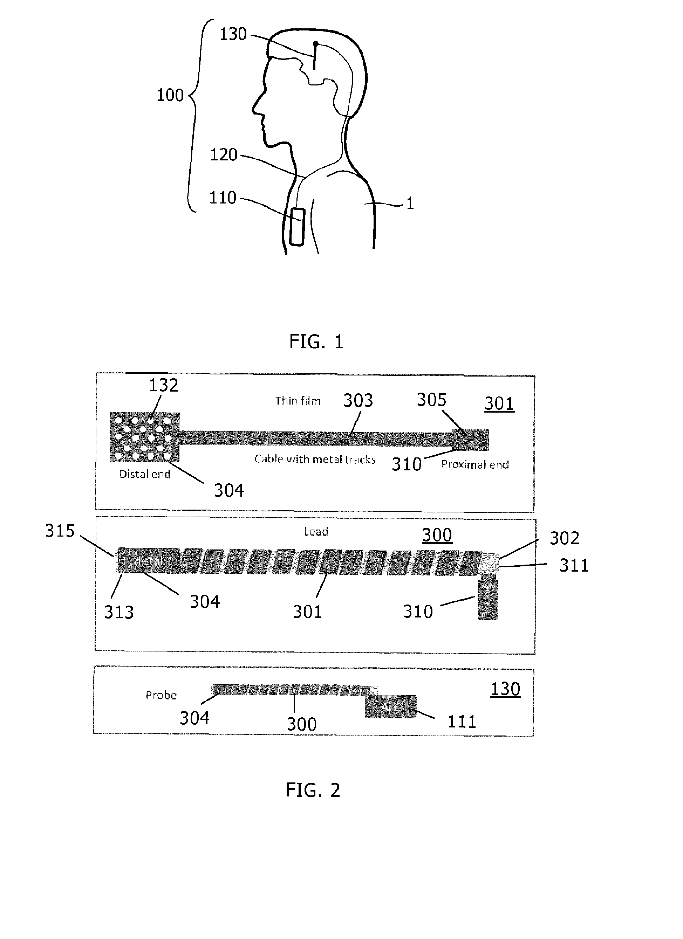

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

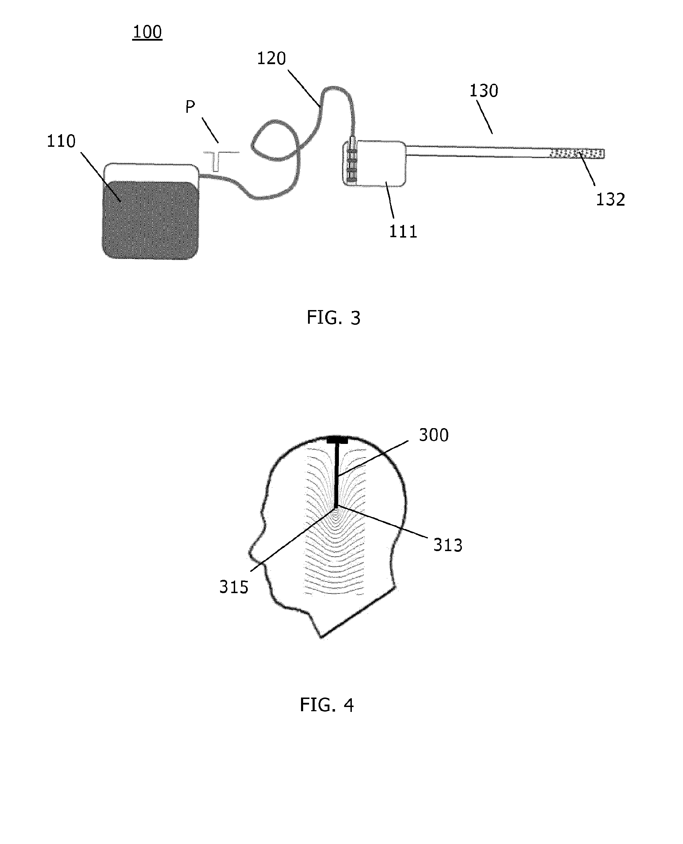

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

Figure 4 shows a schematical drawing of the field enhancement around a conducting lead 300 in a brain in an electric field during an MRI scan. As can be seen in Figure 4, the density of the field lines is increased around the distal tip 315 of the distal end 313 of the lead 300.

As mentioned above, a serious field enhancement may occur as a result of the electrical field during MRI scanning. The density of the field lines at the distal tip 315 of the lead gives rise to tissue heating. As shown in Figure 2, the thin film 301 is wound and is therefore a structure with an inductance. The inductance limits the creation of strong electric fields in the brain during MR scanning and subsequent heating in these areas of the brain. However, field gradient enhancement near the lead 300, for instance at the distal end 313 and the distal tip 315, is still an issue, also because the distal end 313 of the lead 300 is not coiled and therefore it is not an integral part of the inductor. Moreover, the field at the distal lead tip 315 is dominant because the enhancement gradient is most pronounced in this area and most heating of tissue is expected at location in close vicinity of the distal end 313.

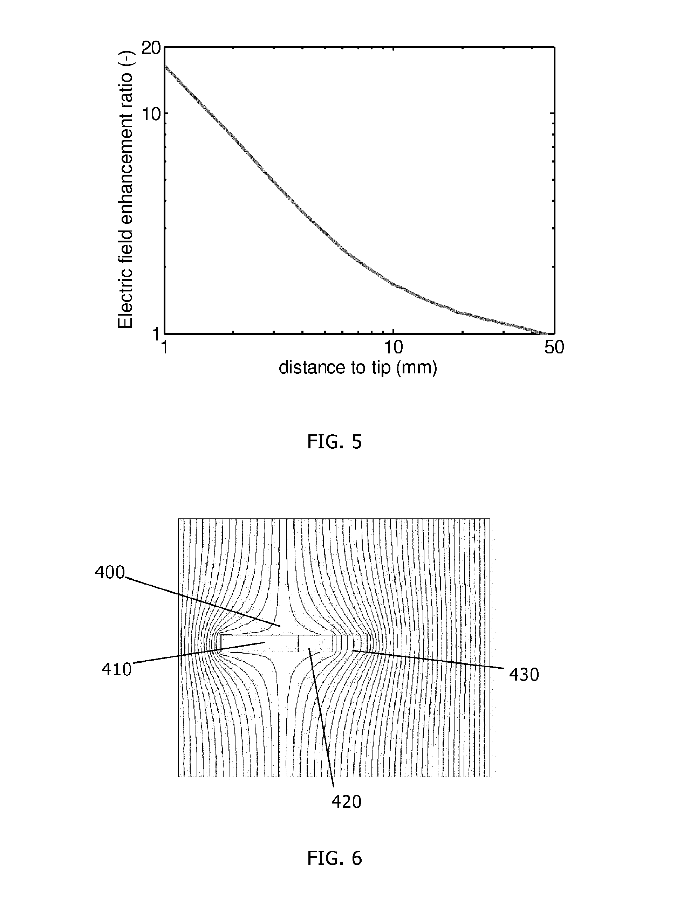

The correlation of this effect is shown in the diagram of Figure 5. This diagram is showing exemplarily a field gradient enhancement simulation at 64 MHz, particularly the correlation of the electric field enhancement ratio and the distance in mm to the distal tip 315 of the lead 300 with logarithmic scaling. As can be seen from the diagram, e.g. the electric field enhancement ratio is decreasing from a value of about 20 to a value below 10, when the distance to the tip is about 2 mm.

Figure 6 illustrates the effect of resistivity and shows vertical equipotential lines of the electric field component. The rectangle-formed bar 400 in the middle is divided into three regions 410, 420, 430 with different conductivities. The left-hand region 410 is highly conductive and has a low resistivity. The equipotential lines cannot enter such a material and thus the region 410 and so they are bent around its shape. Therefore, the density of the equipotential lines at the left-hand side of the bar 400 around region 410 is relatively high. This results in high electric field strength there because the electric field strength is proportional to the density of the equipotential lines or in other words the electric field equals the space derivative of the electric potential field. The right-hand region 430 has a much higher resistance so that equipotential lines can cross this region 430. Because these equipotential lines do not have to bend around the right-hand side anymore, the resulting density of equipotential lines is much lower at the right-hand side of the bar 400 than at the left-hand side. This results in lower electric field strength at the right-hand side, near the region of high resistance, than at the left-hand side, near the region of low resistance.

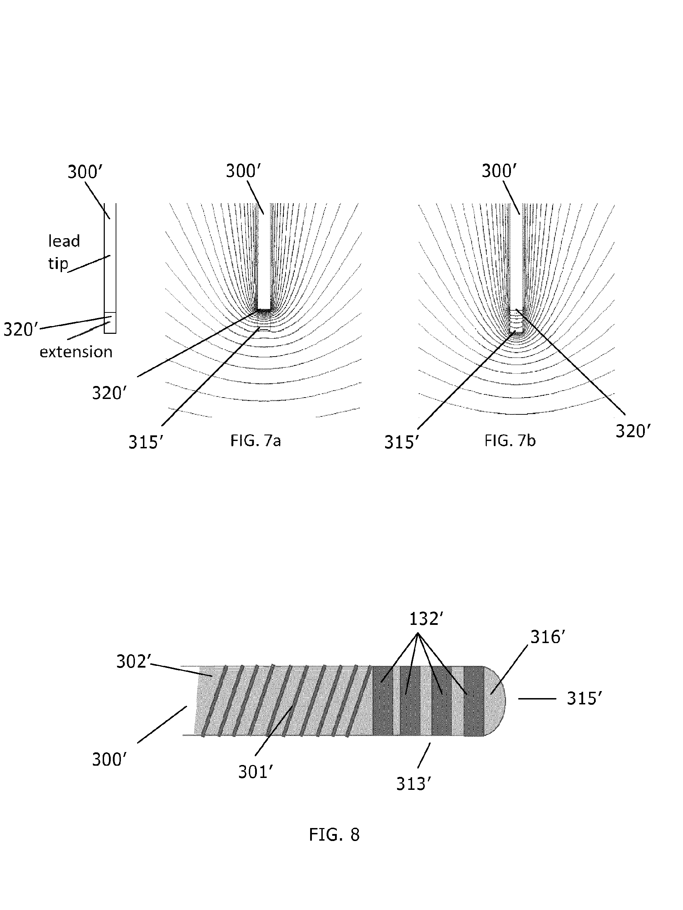

Figure 7a, b show the calculated effect of an extension on the electrical field in the vicinity of the tip 315', 315 of a lead 300', 300 in MRI scanning. As can be seen in Figure 7a, the field around the extension 320' next to the tip 315' of the lead 300' without conductivity is much more pronounced. As shown in Figure 7b, the field around the extension 320 next to the tip 315 of the lead 300 with appropriate conductivity is much smoother and will reduce the risk of heating of the brain tissue during MR scanning operations.

Figure 8 shows the distal section of a classical wire based lead assembly. Such a lead 300' comprises four electrodes 132', which are embedded in a polymer core 302' and arranged at the distal end 313' of the lead 300'. The electrodes 132' are connected to e.g. a main unit or a pulse generator unit via wires 301', which are wound around the polymer core 302'. It is common practice to limit the length of the section 316' of the polymer core 302', which is merely forming the lead tip 315 to less than one millimetre (< 1 mm).

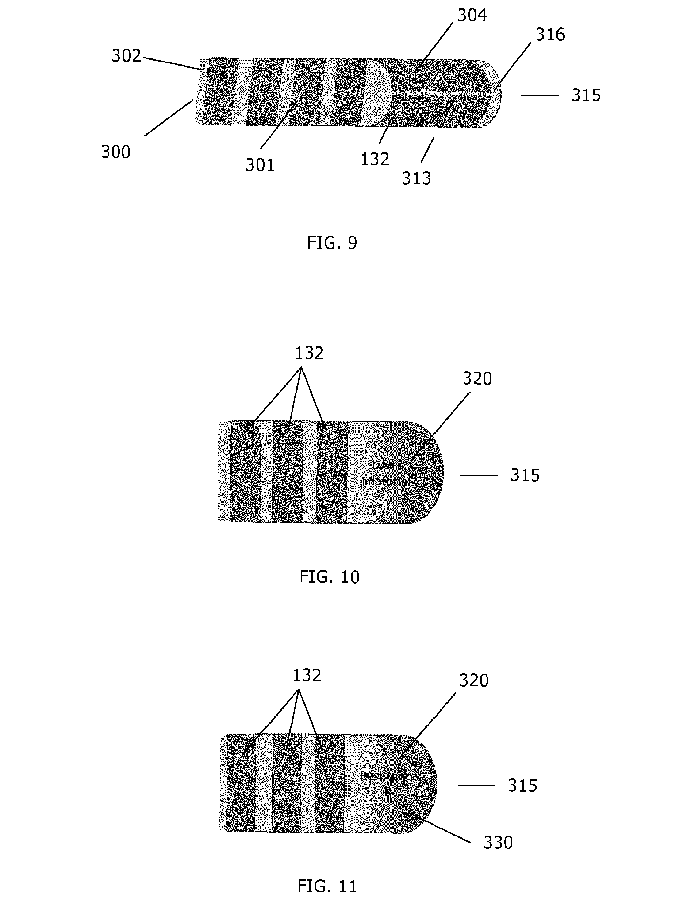

Such a lead 300' may be equipped also with an extension means so as to form a lead according to the present invention. For instance, to the section 316' an extension means 320 as shown in Figures 10 to 14 may be added or the section 316' may be replaced by such an extension means 320.

Figure 9 shows the arrangement for novel, thin film based lead 300. Also in these leads 300 a polymer core 302 as carrier 302 (see also Figure 2) is used to support the electrodes 132 (not shown in detail in Figure 9, see therefore for instance Figure 2) arranged in the distal end 304 of the thin film 301. It is common practice to limit the length of the section 316 of the polymer core 302, which is merely forming the lead tip 315 to less than one millimetre (< 1 mm).

Similarly to the lead 300' of Figure 8, the lead 300 may also be equipped with an extension means so as to form a lead according to the present invention. For instance, to the section 316 an extension means 320 as shown in Figures 10 to 14 may be added or the section 316 may be replaced by such an extension means 320.

Particularly, the lead 300 as shown in Figure 9 comprises a lead tip 315 at the distal end 313 and a plurality of electrodes 132 arranged at the distal end 313 of the lead 300 on the distal end 304 of the thin film 301. The extension means 320 may then be arranged between the plurality of electrodes 132 and the lead tip 315. The extension means 320 may comprise a length of at least 1 mm or more, especially 1.5 mm or more, preferably 2 mm or more. Larger extensions may be more effective than small extensions. A minimum of extension of about 1 mm is required to fully benefit from this effect.

As can be derived from Figures 10 to 13, the extension means 320 is arranged between the most distal electrode of the electrodes 132 and the distal lead tip 315.

As already shown in Figure 5, the field enhancement is most pronounced at the tip end. The simulation in figure 7a, 7b shows that an extension 320 of the tip 315 by means of a low permittivity material (low material) significantly reduces the accumulation of electric field lines near the tip end. The high field concentration occurs in the low permittivity extension material. Here, it does not give rise to heating. Thus and as exemplarily shown in Figure 10, the extension means 320 may have a dielectric constant ε which is configured such that the accumulation of electric field lines near the lead tip 315 is reducible or reduced.

The effectiveness of the extension means 320 can be further enhanced and consequently the size can be reduced by modification of the properties of the core material.

Moreover, an alternative and/or additional feature may be the possibility that the extension means 320 may comprise at least partially a resistive material 330 having an ohmic resistivity (R) as shown in Figure 11.

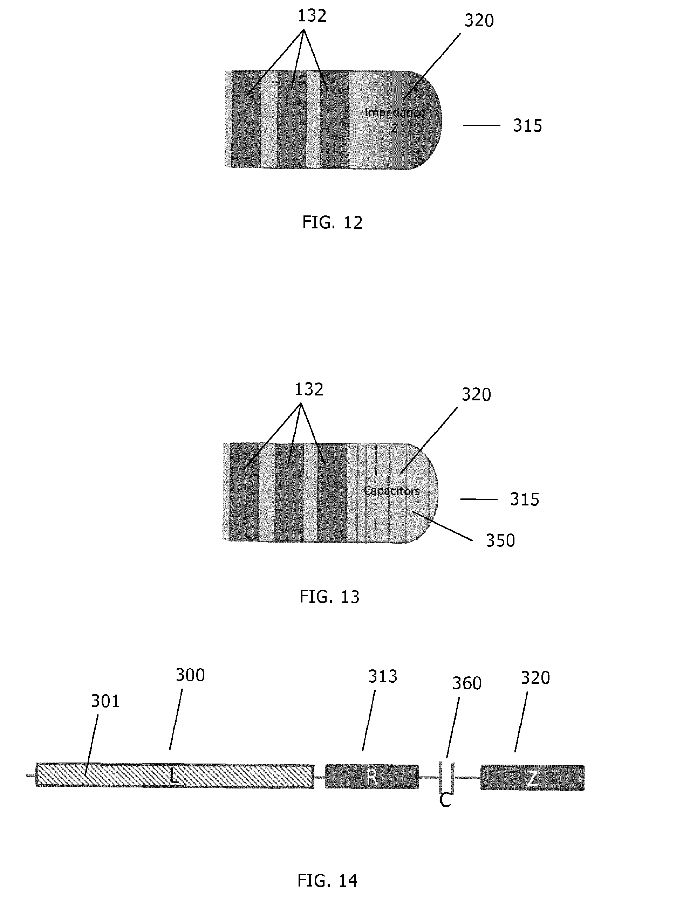

A high impedance Z may further enhance the effectiveness of the extension means 320. This can be seen in Figure 12, where the extension means 320 is configured such that it yields impedance Z when exposed to frequencies used during magnetic resonance imaging. Here, the field around a lead with an extension means 320 with high resistance is calculated. Impedance at MR frequencies can be realized by several means. For instance, ohmic resistivity can be used. A 'high loss' impedance material made of nano materials, such as carbon black in a polymer matrix, can be of benefit here. For instance, the extension means 320 may comprise at least partially particles of conducting material, e.g. nano materials, especially carbon black, whereby preferably the extension means 320 comprises a polymer matrix carrying and/or enclosing the carbon black.

The effect of resistivity in the probe tip extension can be optimized by grading the resistance in the extension means 320, e.g. by a lower impedance part in the vicinity of the distal electrodes 132 and a higher impedance towards the tip 315 of the lead 300.

Alternatively, a capacitive coupling can be used with metals in the tip. As shown in Figure 13, the extension means 320 may comprise at least partially a resistive section having a capacitive resistivity, here by means of a plurality of capacitors 350.

Generally and as schematically shown in Figure 10 to 13, it may be beneficial to use a blunted polymer stylus tip.

The extension means 320 may also comprise at least one capacitive coupling means 360 having a capacitive resistivity. Such a detailed schematic lay-out of the core at the distal-end 313 of the lead 300 shown in Figure 14, which shows a representation of the equivalent electrical circuit. There may be a coiled cable 301' (or a wound thin film 301, which is not shown; compare therefore also with Figure 8) followed in distal direction by the distal end 313 of lead 300. This distal end may have a resistivity R and is capacitively coupled by means of the capacitive coupling means 360 with the extension means 320. This extension means 320 is configured such that it yields impedance Z when exposed to frequencies used during magnetic resonance imaging.

## Claims

1. A lead (300), especially a lead (300) for neural applications, preferably a lead (300) for a neurostimulation and/or neurorecording system, whereby the lead (300) comprises a proximal end (311) and a distal end (313) and whereby the lead (300) comprises at least one extension means (320) at the distal end (313).

2. The lead (300) according to claim 1,
**characterized in that**
the lead (300) comprises a lead tip (315) at the distal end (313) and a plurality of electrodes (132) arranged at the distal end (313) of the lead (300) and that the extension means (320) is arranged between the plurality of electrodes (132) and the lead tip (315).

3. The lead (300) according to any of the preceding claims,
**characterized in that**
the extension means (320) has a length of at least 1 mm or more, especially 1.5 mm or more, preferably 2 mm or more.

4. The lead (300) according to any of the preceding claims,
**characterized in that**
the extension means (320) has a dielectric constant (ε) which is configured such that the accumulation of electric field lines near the lead tip (315) is reducible or reduced.

5. The lead (300) according to any of the preceding claims,
**characterized in that**
the extension means (320) comprises at least partially a resistive material (330) having an ohmic resistivity (R).

6. The lead (300) according to any of the preceding claims,
**characterized in that**
the extension means (320) is configured such that it yields impedance (Z) when exposed to frequencies used during magnetic resonance imaging.

7. The lead (300) according to claim 6,
**characterized in that**
the extension means (320) comprises at least partially particles of conducting material, e.g. nano materials, especially carbon black, whereby preferably the extension means (320) comprises a polymer matrix carrying and/or enclosing the carbon black.

8. The lead (300) according to any of the preceding claims,
**characterized in that**
the extension means (320) comprises at least partially a resistive section having a capacitive resistivity and/or that the extension means (320) comprises at least one capacitive coupling means (360) having a capacitive resistivity.

9. The lead (300) according to claim 8,
**characterized in that**
the extension means (320) comprises a plurality of capacitors (350).

10. The lead (300) according to any of claims 5 to 9,
**characterized in that**
the extension means (320) is arranged and/or configured such that it comprises at least partially a resistivity and/or impedance gradient.

11. The lead (300) according to claim 10,
**characterized in that**
the resistivity and/or impedance gradient is configured such that the extension means (320) comprises at least a first resistivity and/or impedance at the one end of the extension means (320) and at least a second resistivity and/or impedance at a second end of the extension means (320), whereby the first resistivity and/or impedance is higher than the second resistivity and/or impedance.

12. The lead (300) according to claim 11,
**characterized in that**
the first end of the extension means (320) is arranged near to the distal tip (315) of the lead (300) and the second end of the extension means (320) is arranged next to the electrodes (132).

13. The lead (300) according to any of the preceding claims,
**characterized in that**
the lead tip (315) is a blunted polymer stylus tip.

14. A probe (130), especially a probe (130) for neural applications, preferably a probe (130) for a neurostimulation and/or neurorecording system, comprising at least one lead (300) according to any of the preceding claims.

15. A system (100), especially a neurostimulation and/or neurorecording system (100), preferably a deep brain stimulation system (100), comprising at least one lead (300) according to any of claims 1 to 13 and/or at least one probe (130) according to claim 14.
